# EUROPEAN PATENT SPECIFICATION

(11) **EP 1 628 698 B1**
(45) Date of publication and mention of the grant of the patent: **23.07.2008**
(21) Application number: 04734561.6
(22) Date of filing: 24.05.2004
(51) Int. Cl.: A61M 15/00

(54) **MEDICAMENT CONTAINER**
MEDIKAMENTENBEHÄLTER
RECIPIENT A MEDICAMENTS

(30) Priority: 24.05.2003 GB 0312007
(43) Date of publication of application: 01.03.2006
(73) Proprietor: Innovata Biomed Limited, St. Albans AL1 3HW (GB)
(72) Inventor: GORDON, James, Barbourne Worchester WR1 1RB (GB)
(74) Representative: Jones, Stephen Anthony
(86) International application number: PCT/GB2004/002234
(87) International publication number: WO 2004/103446

(56) References cited:
- EP-A- 1 106 196
- CH-A- 588 239
- GB-A- 2 340 758

## Description

This invention relates to a novel form of drug container and/or a method of drug delivery and to medical devices and methods of treatment utilising such containers and/or methods.

Conventionally known powder inhalation devices comprise a medicament housed in a foil covered blister. In use, the foil and the blister are both ruptured, allowing the powdered medicament to be blown or sucked out. However, such systems suffer from the disadvantage that, *inter alia,* powdered medicament can be entrapped in or around the ruptured foil or in the blister space. This can lead to a patient receiving an inconsistent dosage of medicament.

US Patent No. 4,778,054 describes a blister pack, e.g. for housing a powdered medicament, which overcomes or mitigates the disadvantages experienced with prior art blister packs by regulating the ratio of the diameter to depth of each of the blisters.

US Patent No. 5,921,237 to Dura, describes an inhaler comprising a rotatably mounted blister pack disk with means adapted to shear open a sealed blister and thereby deliver the drug dose to the patient.

International Patent Application No. WO 01/43529 - Inhale describes a receptacle for holding fine powders wherein the bottom end of the receptacle is provided with a raised central region that extends upwardly in the cavity of the receptacle. In particular, Inhale describes at page 10, lines 15 to 17 a receptacle which comprises, *inter alia,* a continuously curved wall that forms a raised central region at or near the centre of the receptacle. In use, a powder extraction tube is inserted into the receptacle adjacent or above the raised central region so that a laminar flow occurs and a shear stress is created along the length of the walls of the receptacle.

EP1106196 discloses an inhaler which comprises a blister pack for a powdered medicament. Blister portions are described which have a lid panel and a base panel; the latter having two substantially hemispherical convex portions in which inflow and outflow holes are pricked, and a flow-constriction portion formed between the substantially hemispherical convex portions to define a flow-constriction passage. In use, air flows through the inflow holes to the outflow holes, picking up powdered medicament, and passing through the flow-constriction passage.
Thus, there has long been a need for an improved inhalation system which, in particular, is efficient in emptying a powder receptacle and maximising the effect of the air flow in aerosolising the powder.

It is an aim of embodiments of the present invention to at least partly mitigate the above-mentioned problems.

A novel receptacle and a novel delivery system for powdered medicament have been developed, which provide improved entrainment, deagglomeration and/or aerosolisation of the powder.

In particular, we have surprisingly found a novel receptacle which, comprises a raised diametrical portion and which, in use, is adapted to create laminar flow and shear stress by splitting the air flow into two substantially equal portions.

Thus, according to the invention a first aspect of the present invention there is provided a receptacle for holding a powdered medicament, the receptacle being adapted for engagement with a delivery device and comprising a cavity provided with a raised ridge, characterised in that the raised ridge is adapted for dividing airflow directed, in use, at the raised ridge into two substantially equal parts.

It will be understood by one skilled in the art that the reference to a "ridge" is intended to be distinct from the raised central portion described in WO01/43529.

The ridge is adapted so that, in use, the air flow, in particular the inlet air or flushing air within the receptacle, is split into two substantially equal parts, e.g. providing first and second reservoirs. Thus, the diametrical ridge may be such that the length of the ridge is equivalent to the internal diameter of the receptacle. However, it is within the scope of the invention for the length of the ridge to be less than the length of the internal diameter of the receptacle.

It may be, for example, a radial ridge, that is, a ridge which traverses the radius of the receptacle, or the ridge may traverse a region of the receptacle without contacting the walls of the receptacle. In a further embodiment the ridge, e.g. a radial ridge, may be arcuate, for example, such that the height of the ridge is greater towards the periphery of the receptacle than it is adjacent the middle of the receptacle. This aspect of the invention is advantageous in that, *inter alia,* two vortices are caused along the length of the receptacle. However, in a preferred aspect of the invention, the ridge is substantially diametrical.

The height of the ridge may vary. Thus the height of the ridge may be such that the ridge extends up to the top of the receptacle. However, the ridge will not protrude above the receptacle. Alternatively, the upper surface of the ridge may lie below the top of the receptacle. A ridge which lies beneath the top of the receptacle is preferred so that in use, if desirable, a vent tube and/or inlet tube may be inserted into the receptacle substantially above the ridge.

The raised region acts as a splitter ridge and the gas jet directed at the raised region is divided into two substantially equal portions and after flowing around the edges of the receptacle leaves the receptacle at substantially the same velocity as it entered.

Apparatus adapted for the entrainment, and/or aerosolisation of a medicament may comprise a housing having a holder that is adapted to receive a receptacle as hereinbefore described.

The receptacle will generally be provided with a cover, e.g. a frangible sealing member, such as a foil strip. Preferably, the apparatus also comprises a piercing mechanism that is adapted to pierce a hole in the receptacle cover. Preferably the piercing mechanism is adapted to pierce a hole in the top of this cover, that is, away from the base of the receptacle. Alternatively, the apparatus may comprise means for complete or partial removal of the cover. The apparatus may also include an extraction tube, e.g. a powder extraction tube, that is adapted to be placed into the receptacle after the cover has been pierced or removed. The extraction tube may itself act as the piercing member or a separate piercing member and extraction tube may be provided. In use the extraction tube may be positioned above the diametrical ridge, although it is within the scope of the present invention that the extraction tube may be displaced from the ridge.

In the embodiment of the invention in which the ridge is a substantially radial ridge, the vent tube is preferentially placed substantially above the ridge, whilst the extraction tube is preferentially placed remote from the ridge, i.e. substantially coaxial with the ridge, but in the ridgeless region of the receptacle.

Furthermore, in addition to the piercing mechanism, the apparatus may include a vent forming mechanism, e.g. for forming a vent, i.e. an air or gas inlet, or multiple vents, in the top end of the receptacle.

According to a further aspect of the present invention there is also provided a plurality of dosage units arranged in series, each unit being as hereinbefore and/or hereinafter described. The units may be releasably or permanently attached to one another so as to be in a chain-like conformation, preferably a flexible or semi-flexible chain. The design of dosage units in accordance with the invention makes such flexibility possible.

A series of dosage units in accordance with this aspect of the invention is advantageous for use in an inhaler, because it allows sequential presentation of doses of a medicament to the inhalation passage of the inhaler as the series is indexed through the inhaler. If the series is in the form of a flexible chain, it can then be rolled or folded up for compact storage in the inhaler. The series may be of any appropriate length. It may, for instance, be supplied in a length greater than might be needed for use in an inhaler, but capable of being broken up into usable lengths. In an especially preferred embodiment the plurality of dosage units are contained in a cartridge and such a cartridge forms a further aspect of the invention.

According to a still further aspect of the present invention there is also provided a powdered medicament delivery device comprising a dosage unit as hereinbefore described.

The medicament is a powder and therefore, preferably, the delivery device is an inhaler, e.g. a dry powder inhaler.

When the medicament delivery device comprises an inhaler the medicament channel of the rupturing member may comprise an air channel and/or an aerosolisation channel. Whilst, generally, the medicament/air channel in the rupturing member is adapted for the removal of medicament, e.g. in aerosolised form, from the metering member, it may also be used to introduce, e.g. flushing air in the receptacle.

In the inhaler of the invention the medicament dosage units are preferably presented in a cartridge as hereinbefore described.

The term dry powder should be construed as meaning a variety of aerosolisable materials, thus, for example, conventional powders, spray dried materials, such as granules, etc.

The receptacle of the invention is preferentially adapted to be a drug reservoir, e.g. housing a powdered medicament. A variety of medicaments may be administered by using the inhaler of the invention. Such medicaments are generally suitable for the treatment of asthma, COPD and respiratory infections. Such medicaments include, but are not limited to β₂-agonists, e.g. fenoterol, formoterol, pirbuterol, reproterol, rimiterol, salbutamol, salmeterol and terbutaline; non-selective beta-stimulants such as isoprenaline ; xanthine bronchodilators, e.g. theophylline, aminophylline and cholinetheophyllinate ; anticholinergics, e. g. ipratropium bromide; mast cell stabilisers, e.g. sodium cromoglycate and ketotifen ; bronchial anti-inflammatory agents, e.g. nedocromil sodium; and steroids, e.g. beclomethasone dipropionate, fluticasone, budesonide, flunisolide and ciclesonide, and isomers and/or salts or derivatives thereof.

Specific combinations of medicaments which may be mentioned include combinations of steroids, such as, beclomethasone dipropionate and formoterol ; beclomethasone dipropionate and salmeterol; fluticasone and formoterol ; fluticasone and salmeterol; budesonide and formoterol ; budesonide and salmeterol; flunisolide and formoterol; and flunisolide and salmeterol. It is also within the scope of this invention to include combinations of one or more of the aforementioned steroids with one or more of the aforementioned β₂-agonists. When the receptacle of the invention is used in conjunction with a combination therapy as hereinbefore described, each of the first and second reservoirs may hold a combination therapy. However, in one option of the invention, the first reservoir may contain a first medicament and the second reservoir may contain a second medicament.

Further medicaments which may be mentioned include systemically active materials, such as, proteinaceous compounds and/or macromolecules, for example, hormones and mediators, such as insulin, human growth hormone, leuprolide and alpha interferon; growth factors, anticoagulants, immunomodulators, cytokines and nucleic acids.

The invention will now be described by way of example only and with reference to the accompanying drawings in which,
Figures 1 and 2 are representations of a prior art system comprising a receptacle with a raised central region;
Figure 3 is a front cross-section of a receptacle of the invention;
Figure 4 is a front cross-sectional view of a receptacle containing medicament powder;
Figure 5 is a side cross-section of a receptacle of the invention;
Figure 6 is a side cross-sectional view of a receptacle containing medicament;
Figure 7 is a front cross-section of a receptacle comprising a radial ridge;
Figure 8 is a side cross-sectional view of a receptacle comprising a radial ridge;
Figure 9 is a front cross-sectional view of a receptacle with air inlet tube inserted;
Figure 10 is a front cross-sectional view of a receptacle of the invention showing air flow;

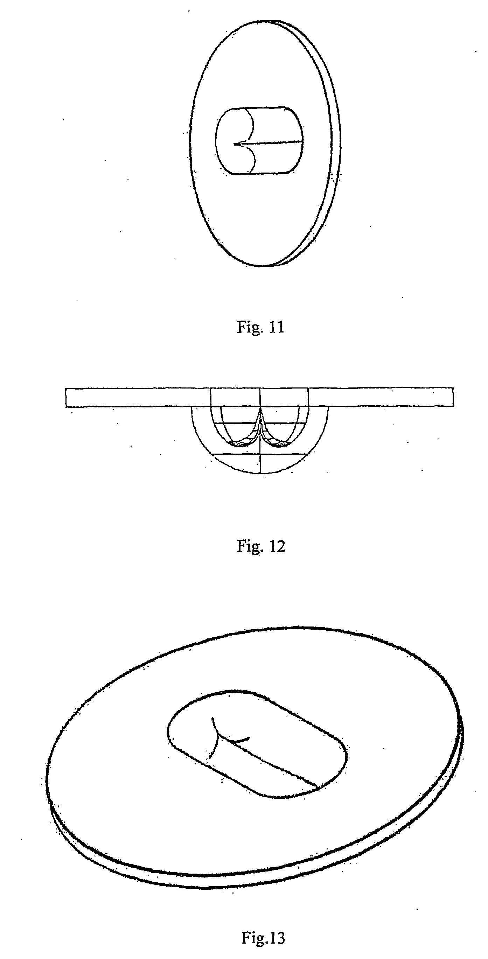

Figure 11 is a perspective representation of a receptacle of the invention;
Figure 12 is a schematic representation;
Figure 13 is a perspective representation from above;
Figure 14 is a perspective representation with vent tubes inserted;
Figure 15 is a schematic representation of figure 14;
Figure 16 is a side cross-sectional view of a receptacle comprising a partial radial ridge;
Figure 17 is a side cross-sectional view of a receptacle comprising a partial radial ridge with inlet and outlet tubes inserted;
Figure 18 illustrates a bent inlet tube;
Figure 19 also illustrates a bent inlet tube;
Figure 20 illustrates how an inlet or extraction tube may be bent;
Figure 21 illustrates a bent extraction tube; and
Figure 22 illustrates a series of receptacles.

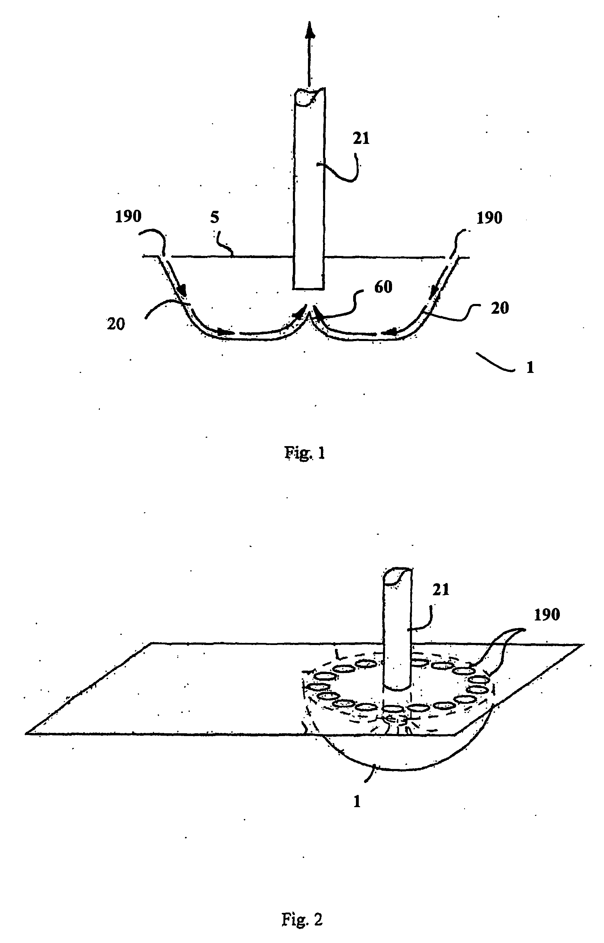

Referring to figures 1 and 2, the prior art comprises a receptacle (1) with a raised central region (60). In use, a powder outlet tube (21) is inserted in the receptacle above the raised central region (60). Vents (air inlets) (190) are pierced in the foil covering (5) around the periphery of the receptacle (1). Air (20) flows in through the vents, picking up powder and exits through the central vent tube (21).

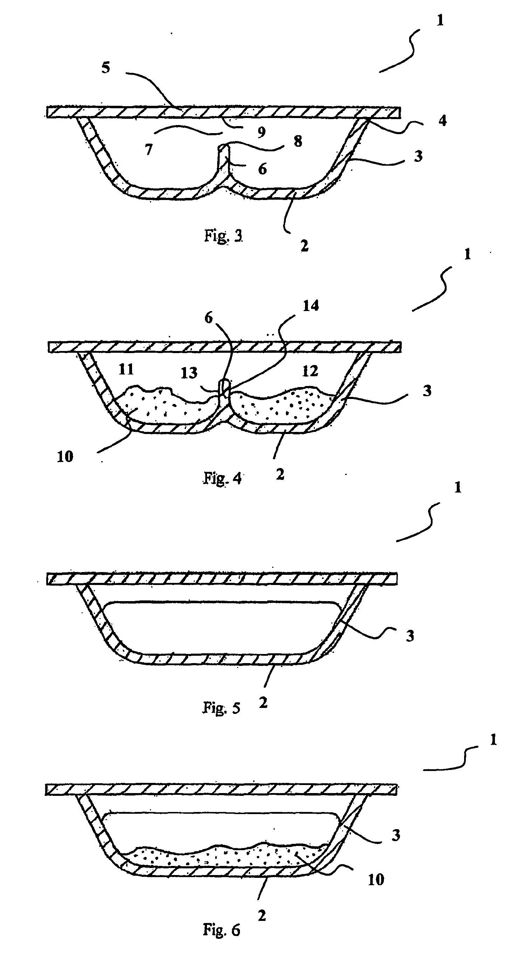

Referring to figures 3 to 6, a receptacle (1) of the invention comprises a container with a base (2) and side walls (3). The end (4) of the side walls (3) distal from the base (2), support a foil cover (5). The base (2) is provided with a raised diametrical ridge (6). The height of the ridge (6) is such that space (7) exists between the top (8) of the ridge (6) and the underside (9) of the cover (5). Medicament powder (10) is provided on the first (13) and second (14) sides of the ridge (6). The ridge (6) essentially divides the receptacle (1) into two separate compartments (11 and 12).

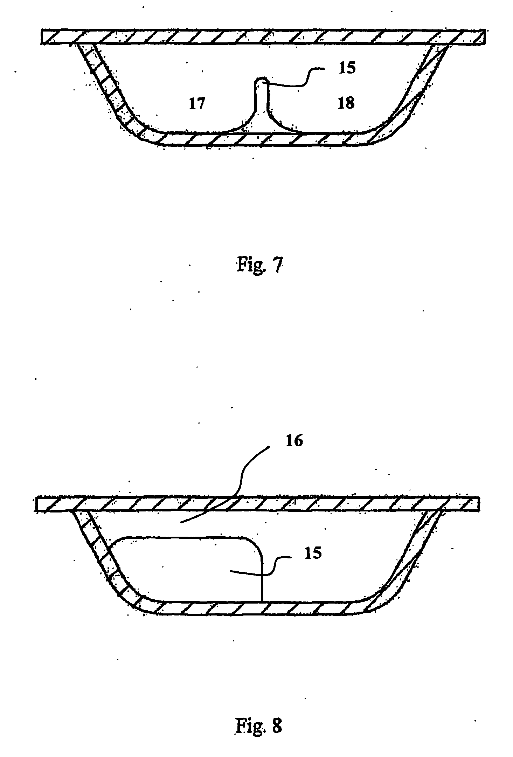

Referring to figures 7 and 8, the ridge (15) comprises a radial element such that only a portion (16) of the receptacle (1) is divided in separate components (17 and 18).

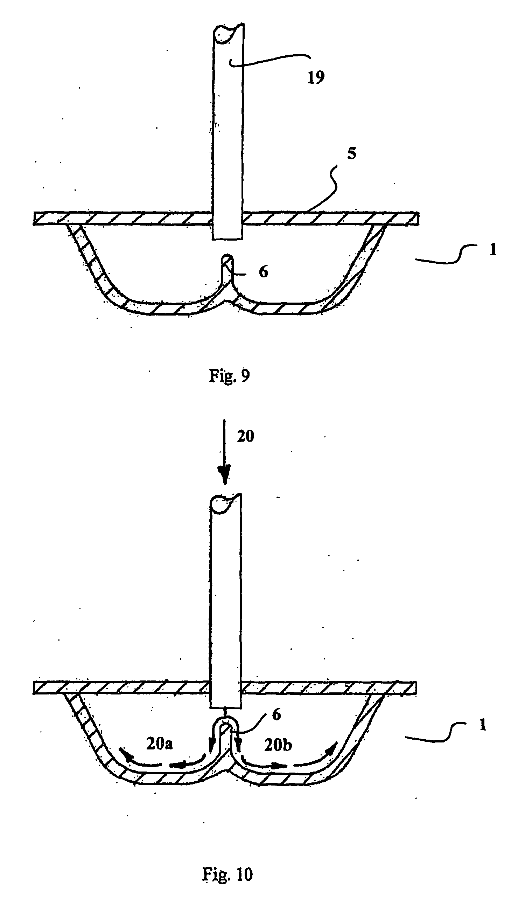

Referring to figures 9 and 10, in use an air inlet conduit (19) is inserted through the cover (5) into the receptacle (1). The inlet (19) rests substantially above the ridge (6). Inlet air (20) is passed through the inlet conduit (19) and upon hitting the ridge (6), the flowing air (20) is divided into two substantially equal parts (20a and 20b). An outlet conduit (not shown) is provided, through which the inlet air and aerosolised powder/medicament escapes.

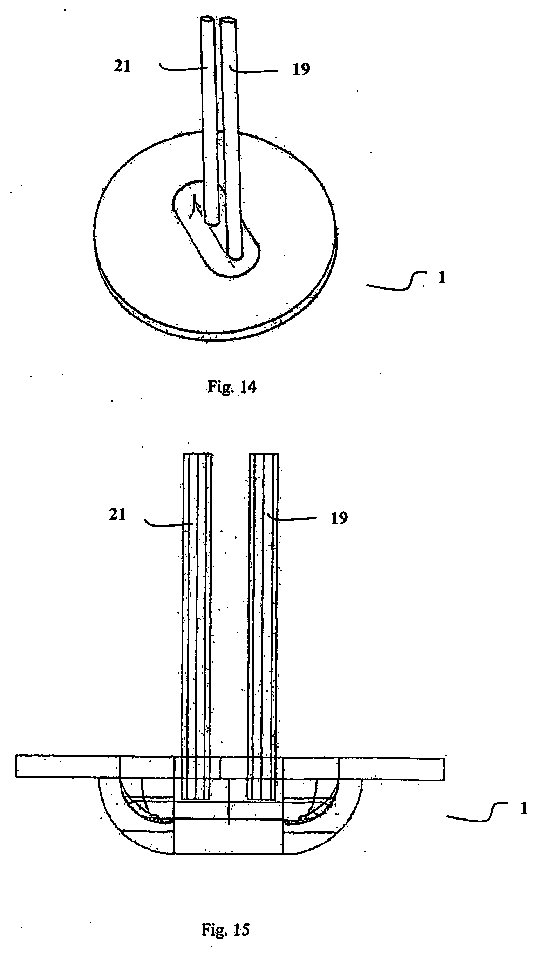

Referring to figures 14 and 15, the receptacle (1) is shown with air inlet (19) and outlet (21).

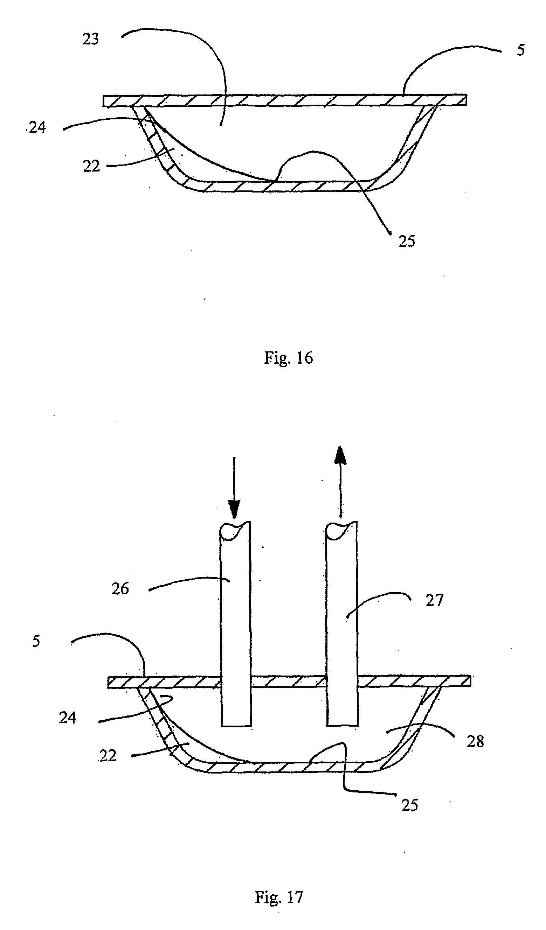

Referring to figure 16, the ridge (22) comprises a partial radial element such that only a portion (23) of the receptacle (1) is divided in separate compartments. Furthermore, the ridge (22) is arcuate such that the height of the ridge is greater towards the periphery (24) of the receptacle than it is adjacent the middle (25) of the receptacle.

Referring to figure 17, an (air) inlet tube (26) is inserted through the cover (5), adjacent the ridge (22), whilst an (powder) outlet tube (27) is inserted adjacent the ridge free portion (28) of the receptacle (1). This aspect of the invention is advantageous in that, *inter alia,* two vortices are caused along the length of the receptacle.

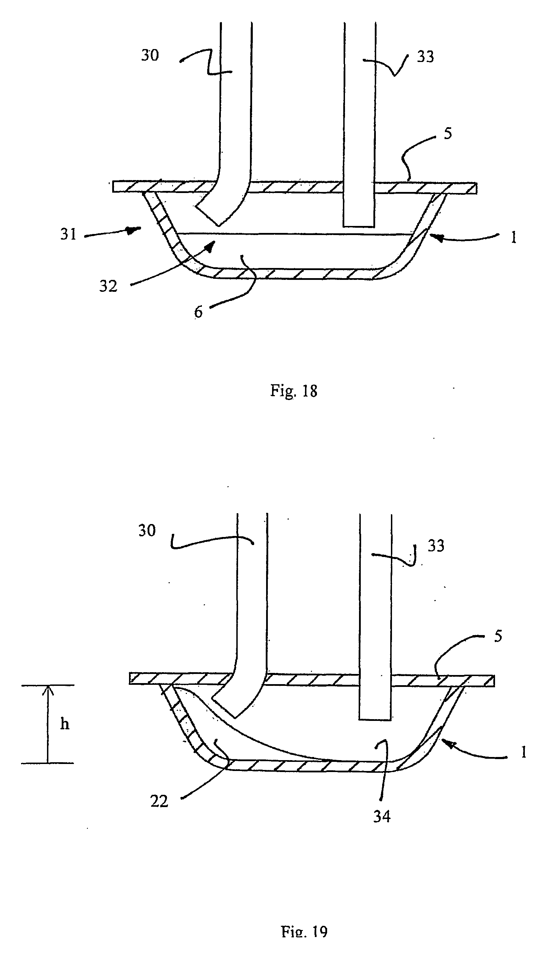

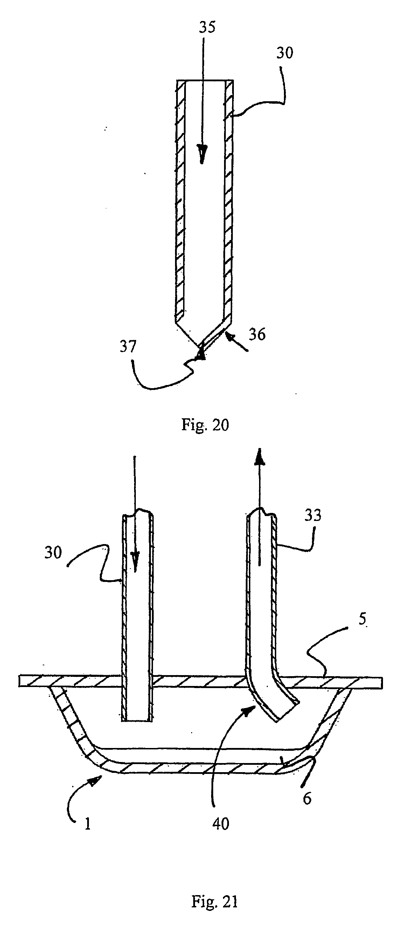

Figure 18 illustrates a further embodiment of the present invention in which the inlet tube 30 is bent at a lower region. This lower region forms a piercing portion of the inlet tube as the tube is urged against the foil cover 5 when the medicament stored in the receptacle 1 is to be dispensed. The angle of curvature defining how bent the lower portion of the inlet tube 30 is may be between 0° and 90°. It will be understood that when the angle of curvature is such that piercing of the foil cover may be impeded by the curved outer portion of the tube, a piercing point may be fabricated on the tube or the tube may be fabricated as shown more clearly in figure 20.

The curvature at the lower portion of the inlet tube is such that when air or other dispensing gas is produced at the inlet, the air entering the receptacle is directed against the side wall region 31 as well as a portion 32 of the ridge 6. As a result swirl flow of air is induced within the receptacle which helps to entrain medicament in the receptacle drawing it towards the outlet tube 33.

Figure 19 illustrates a further embodiment of the present invention in which the ridge 22 is an arcuate ridge having a height h above an inner surface 34 of the receptacle which is higher at the edge of the receptacle than elsewhere. As illustrated in figure 19 the inlet 30 is curved at a lower portion so that air entering the receptacle is incident upon the side walls and a portion of the ridge 22. This helps create an advantageous air flow within the receptacle which helps gather up medicament located in the receptacle and move it towards the extraction (or outlet) tube 33.

Figure 20 illustrates how air flow may be directed from a non-axial direction whilst still retaining the piercing ability of either an inlet 30 or outlet 33 tube. Air or other gas is input at the inlet 35 and is directed at an angle away from the axial flow by a bent portion of the tube 30. The bent portion 36 has a piercing tip 37 which helps the tube penetrate the foil or other covering material which seals the receptacle. The bent portion 36 may be aligned at a desired angle so that the air flow inwards to a receptacle during use either flows in a direction towards the base of the receptacle or towards a side wall.

Figure 21 illustrates a still further embodiment of the present invention in which an outlet tube 33 has a lower region 40 which is bent. In this embodiment the inlet tube 30 is substantially linear so that air input at the inlet is directed substantially at right angles to the base of the receptacle. The inlet tube is directed so that it is over the ridge 6 which helps split the incoming air into two equal portions. Splitting the air in this manner helps conserve momentum of the incoming air as will be understood by those skilled in the art.

It will be understood that according to embodiments of the present invention both or either of the inlet and extraction tubes may be angled at any angle from 0 (in which the lower region of the tube is substantially perpendicular to the base of the receptacle) and 90° (in which the piercing end region of the tube is substantially parallel with the base of the receptacle. It will be understood that the ratio of receptacle diameter to jet diameter (the diameter of the inlet or extraction tube) may be selected to determine characteristics of the air flow within the receptacle.

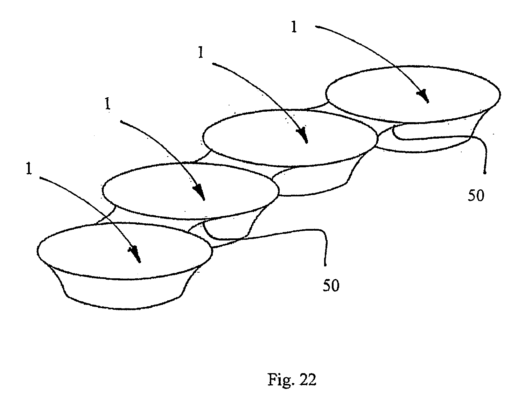

Figure 22 illustrates a series of receptacles 1 which are held together by a web 50. The receptacles may be formed from plastics material or may be pressed from a single aluminium sheet. As an alternative independent receptacles may be provided for use in a delivery device as appropriate.

It will be understood that according to embodiments of the present invention the manner in which the inlet and outlet tubes may be bent may be selected to provide desirable air flow in the receptacle during use. In a preferable embodiment the inlet tube is turned towards the outside of the receptacle so that air flow impacts upon that side and is split in two by the ridge. The extraction tube is likewise turned towards an edge region of the receptacle but at 180° to the direction in which the inlet tube is turned. As a result the air path length within the receptacle is maximised. This makes sure that all medication is cleared out of the blister and provides a maximum opportunity for turbulent air flow to interact with medicament particles so as to deagglomerate those particles. As an alternative either or both of the tubes may be turned inwardly towards a central region of the receptacle.

The above specific examples have been described by way of example only. It will be understood that modifications may be made to these without departing from the scope of the present invention.

## Claims

1. A receptacle (1) for holding a powdered medicament, the receptacle (1) being adapted for engagement with a delivery device and comprising a cavity provided with a raised ridge (6; 15; 22), **characterised in that** the raised ridge (6; 15; 22)is adapted for dividing airflow directed, in use, at the raised ridge (6; 15; 22) into two substantially equal parts (20a and 20b).

2. A receptacle (1) according to claim 1 **characterised in that** the raised ridge (15) traverses the radius of the receptacle (1).

3. A receptacle (1) according to claims 1 or 2 **characterised in that** the raised ridge (22) is an arcuate ridge.

4. A receptacle (1) according to claim 1 **characterised in that** the ridge traverses a region of the receptacle without contacting the walls (3) of the receptacle (1).

5. A receptacle (1) according to claim 1 **characterised in that** the ridge (6) is substantially diametrical.

6. A receptacle (1) according to any preceding claim **characterised in that** the height of the ridge (6; 15; 22) is such that the ridge (6; 15; 22) extends substantially to the top of the receptacle (1).

7. A receptacle (1) according to any one of claims 1 to 5 **characterised in that** the upper surface (8) of the ridge (6) lies below the top of the receptacle (1).

8. A receptacle according to any preceding claim **characterised in that** a frangible sealing member (5) is fixed to the upper part of the receptacle (1).

9. A cartridge or strip comprising a series of receptacles (1) according to claim 1.

10. A delivery device for powdered medicament, said device comprising a receptacle (1) according to claim 1, and the delivery device including means for, in use, directing airflow at the raised ridge (6; 15; 22) of the receptacle (1) such that the airflow is divided by the raised ridge (6; 15; 22) into two substantially equal parts (20a and 20b).

11. A delivery device according to claim 10 **characterised in that** the delivery device includes means (19; 21; 26; 27; 30; 33) for rupturing a frangible cover (5).

12. A delivery device according to claim 10 or claim 11 **characterised in that** the means for directing airflow at the raised ridge (6; 15; 22) comprises an air inlet tube (19; 26; 30).

13. A delivery device according to any one of claims 10 to 12 **characterised in that** the delivery device also includes an extraction tube (21; 27; 33).

14. A delivery device according to claim 13 **characterised in that** the extraction tube (21; 27; 33) and/or the air inlet (19; 26; 30) acts as the piercing member.

15. A delivery device according to claim 13 or claim 14 **characterised in that** in use the extraction tube (21; 27; 33) is positioned above the ridge (6; 15; 22).

16. A delivery device according to any one of claims 10 to 15 **characterised in that** the delivery device is a dry powder inhaler.

17. A delivery device according to claim 16 **characterised in that** the inhaler comprises a plurality of medicament receptacles (1).

18. A delivery device according to claim 17 **characterised in that** each receptacle (1) houses two medicaments.

19. A delivery device according to claim 18 **characterised in that** each medicament is held at either side of the ridge (6; 15; 22).

20. A receptacle (1) according to any one of claims 1 to 8, **characterised in that** the powder is selected from the group of drugs for the treatment of asthma, COPD or respiratory infections such as β₂-agonists, e.g. fenoterol, formoterol, pirbuterol, reproterol, rimiterol, salbutamol, salmeterol and terbutaline; non-selective beta-stimulants such as isoprenaline; xanthine bronchodilators, e.g. theophylline, aminophylline and choline theophyllinate; anticholinergics, e.g. ipratropium bromide; mast cell stabilisers, e.g. sodium cromoglycate and ketotifen; bronchial anti-inflammatory agents, e.g. nedocromil sodium; and steroids, e.g. beclomethasone dipropionate, fluticasone, budesonide, flunisolide and ciclesonide, and isomers and/or salts or derivatives thereof.

21. A receptacle (1) according to claim 20 **characterised in that** receptacle (1) comprises a combination of medicaments, selected from steroids, such as, beclomethasone dipropionate and formoterol; beclomethasone dipropionate and salmeterol; fluticasone and formoterol; fluticasone and salmeterol; budesonide and formoterol; budesonide and salmeterol; flunisolide and formoterol; and flunisolide and salmeterol,

22. A receptacle (1) according to any one of claims 1 to 8 **characterised in that** the receptacle (1) comprises a systemically active medicament, such as, proteinaceous compounds and/or macromolecules, for example, hormones and mediators, such as insulin, human growth hormone, leuprolide and alpha interferon; growth factors, anticoagulants, immunomodulators, cytokines and nucleic acids.

## Patentansprüche

1. Behälter (1) zur Aufnahme eines pulverisierten Medikaments, wobei der Behälter (1) für das Zusammenfügen mit einer Zuführvorrichtung angepasst ist und einen mit einem erhabenen Grat (6; 15; 22) versehenen Hohlraum umfasst, **dadurch gekennzeichnet, dass** der erhabene Grat (6; 15; 22) so angepasst ist, dass er einen bei Verwendung auf den erhabenen Grat (6; 15; 22) gerichteten Luftstrom in zwei im Wesentlichen gleiche Teile (20a und 20b) teilt.

2. Behälter (1) nach Anspruch 1, **dadurch gekennzeichnet, dass** der erhabene Grat (15) den Radius des Behälters kreuzt

3. Behälter (1) nach den Ansprüchen 1 oder 2, **dadurch gekennzeichnet, dass** der erhabene Grat (22) ein bogenförmiger Grat ist.

4. Behälter (1) nach Anspruch 1, **dadurch gekennzeichnet, dass** der Grat einen Bereich des Behälters kreuzt, ohne die Wände (3) des Behälters (1) zu kontaktieren.

5. Behälter (1) nach Anspruch 1, **dadurch gekennzeichnet, dass** der Grat (6) im Wesentlichen diametrisch ist.

6. Behälter (1) nach einem vorangehenden Anspruch, **dadurch gekennzeichnet, dass** die Höhe des Grats (6; 15; 22) so ist, dass sich der Grat (6; 15; 22) im Wesentlichen zur Oberseite des Behälters (1) erstreckt.

7. Behälter (1) nach einem der Ansprüche 1 bis 5, **dadurch gekennzeichnet, dass** die obere Oberfläche (8) des Grats (6) unter der Oberseite des Behälters (1) liegt.

8. Behälter nach einem vorangehenden Anspruch, **dadurch gekennzeichnet, dass** am oberen Teil des Behälters (1) ein zerbrechliches Dichtungselement (5) angebracht ist.

9. Patrone oder Streifen, die/der mehrere Behälter (1) nach Anspruch 1 umfasst.

10. Zuführvorrichtung für pulverisierte Medikamente, wobei die Vorrichtung einen Behälter (1) nach Anspruch 1 umfasst und die Zuführvorrichtung Mittel einschließt, um bei Verwendung einen Luftstrom am erhabenen Grat (6; 15; 22) des Behälters (1) so zu lenken, dass der Luftstrom durch den erhabenen Grat (6; 15; 22) in zwei im Wesentlichen gleiche Teile (20a und 20b) geteilt wird.

11. Zuführvorrichtung nach Anspruch 10, **dadurch gekennzeichnet, dass** die Zuführvorrichtung Mittel (19; 21; 26; 27; 30; 33) zum Zerbrechen einer zerbrechlichen Abdeckung (5) umfasst.

12. Zuführvorrichtung nach Anspruch 10 oder Anspruch 11, **dadurch gekennzeichnet, dass** die Mittel zum Richten eines Luftstroms auf den erhabenen Grat (6; 15; 22) einen Lufteinlassschlauch (19; 26; 30) umfassen.

13. Zuführvorrichtung nach einem der Ansprüche 10 bis 12, **dadurch gekennzeichnet, dass** die Zuführvorrichtung auch einen Entnahmeschlauch (21; 27; 33) umfasst.

14. Zuführvorrichtung nach Anspruch 13, **dadurch gekennzeichnet, dass** der Entnahmeschlauch (21; 27; 33) und/oder der Lufteinlass (19; 26; 30) als Durchstechelement dient/dienen.

15. Zuführvorrichtung nach Anspruch 13 oder Anspruch 14, **dadurch gekennzeichnet, dass** bei Verwendung der Entnahmeschlauch (21; 27; 33) oberhalb des Grats (6; 15; 22) positioniert ist.

16. Zuführvorrichtung nach einem der Ansprüche 10 bis 15, **dadurch gekennzeichnet, dass** die Zuführvorrichtung ein Trockenpulverinhalator ist.

17. Zuführvorrichtung nach Anspruch 16, **dadurch gekennzeichnet, dass** der Inhalator eine Mehrzahl von Medikamentenbehältern (1) umfasst.

18. Zuführvorrichtung nach Anspruch 17, **dadurch gekennzeichnet, dass** jeder Behälter (1) zwei Medikamente enthält.

19. Zuführvorrichtung nach Anspruch 18, **dadurch gekennzeichnet, dass** jedes Medikament auf jeweils einer Seite des Grats (6; 15; 22) aufgenommen wird.

20. Behälter (1) nach einem der Ansprüche 1 bis 8, **dadurch gekennzeichnet, dass** das Pulver ausgewählt ist aus der Arzneimittelgruppe zur Behandlung von Asthma, COPD oder Atemwegsinfektionen, wie z. B. β₂-Agonisten, z. B. Fenoterol, Formoterol, Pirbuterol, Reproterol, Rimiterol, Salbutamol, Salmeterol und Terbutalin, nicht selektive Beta-Stimulanzien wie z. B. Isoprenalin, Xanthin-Bronchodilatatoren, z. B. Theophyllin, Aminophyllin und Cholintheophyllinat, Anticholinergika, z. B. Ipratropiumbromid, Mastzellenstabilisatoren, z. B. Natriumchromoglycat und Ketotifen, brochialentzündungshemmende Mittel, z. B. Nedocromilnatrium, und Steroide, z. B. Beclomethasondipropionat, Fluticason, Budesonid, Flunisolid und Ciclesonid, und Isomere und/oder Salze oder Derivate derselben.

21. Behälter (1) nach Anspruch 20, **dadurch gekennzeichnet, dass** der Behälter (1) eine Kombination von Medikamenten umfasst, ausgewählt aus Steroiden, wie z. B. Beclomethasondipropionat und Formoterol, Beclomethasondipropionat und Salmeterol, Fluticason und Formoterol, Fluticason und Salmeterol, Budesonid und Formoterol, Budesonid und Salmeterol, Flunisolid und Formoterol und Flunisolid und Salmeterol.

22. Behälter (1) nach einem der Ansprüche 1 bis 8, **dadurch gekennzeichnet, dass** der Behälter (1) ein systemisch wirksames Medikament umfasst, wie z. B. Proteinverbindungen und/oder -makromoleküle, beispielsweise Hormone und Mediatoren, wie z. B. Insulin, menschliches Wachstumshormon, Leuprolid und Alpha-Inferon, Wachstumsfaktoren, Antikoagulanzien, Immunmodulatoren Cytokine und Nukleinsäuren.

## Revendications

1. Réceptacle (1) destiné à contenir un médicament en poudre, le réceptacle (1) étant prévu pour un engagement avec un dispositif d'administration et comportant une cavité pourvue d'une nervure relevée (6 ; 15 ; 22), **caractérisé en ce que** la nervure relevée (6 ; 15 ; 22) est prévue pour diviser un écoulement d'air dirigé, lors de l'utilisation, au niveau de la nervure relevée (6 ; 15 ; 22) en deux parties sensiblement égales (20a et 20b).

2. Réceptacle (1) selon la revendication 1, **caractérisé en ce que** la nervure relevée (15) traverse le rayon du réceptacle (1).

3. Réceptacle (1) selon la revendication 1 ou 2, **caractérisé en ce que** la nervure relevée (22) est une nervure courbe.

4. Réceptacle (1) selon la revendication 1, **caractérisé en ce que** la nervure traverse une zone du réceptacle sans venir en contact avec les parois (3) du réceptacle (1).

5. Réceptacle (1) selon la revendication 1, **caractérisé en ce que** la nervure (6) est sensiblement diamétrale.

6. Réceptacle (1) selon l'une quelconque des revendications précédentes, **caractérisé en ce que** la hauteur de la nervure (6 ; 15 ; 22) est telle que la nervure (6 ; 15 ; 22) s'étend sensiblement jusqu'au sommet du réceptacle (1).

7. Réceptacle (1) selon l'une quelconque des revendications 1 à 5, **caractérisé en ce que** la surface supérieure (8) de la nervure (6) s'étend sous le sommet du réceptacle (1).

8. Réceptacle selon l'une quelconque des revendications précédentes, **caractérisé en ce qu'**un élément d'obturation pouvant être rompu (5) est fixe sur la partie supérieure du réceptacle (1).

9. Cartouche ou bande comportant une série de réceptacles (1) selon la revendication 1.

10. Dispositif d'administration pour médicament en poudre, ledit dispositif comportant un réceptacle (1) selon la revendication 1, et le dispositif d'administration comprenant des moyens destinés, lors de l'utilisation, à diriger un écoulement d'air au niveau de la nervure relevée (6 ; 15 ; 22) du réceptacle (1) de telle sorte que l'écoulement d'air est divisé par la nervure relevée (6 ; 15 ; 22) en deux parties sensiblement égales (20a et 20b).

11. Dispositif d'administration selon la revendication 10, **caractérisé en ce que** le dispositif d'administration comprend des moyens (19 ; 21 ; 26 ; 27 ; 30 ; 33) destiné à rompre un couvercle pouvant être rompu (5).

12. Dispositif d'administration selon la revendication 10 ou la revendication 11, **caractérisé en ce que** les moyens destinés à diriger un écoulement d'air au niveau de la nervure relevée (6 ; 15 ; 22) comportent un tube d'entrée d'air (19 ; 26 ; 30).

13. Dispositif d'administration selon l'une quelconque des revendications 10 à 12, **caractérisé en ce que** le dispositif d'administration comprend également un tube d'extraction (21 ; 27 ; 33).

14. Dispositif d'administration selon la revendication 13, **caractérisé en ce que** le tube d'extraction (21 ; 27 ; 33) et/ou l'entrée d'air (19 ; 26 ; 30) agit en tant qu'élément de perçage.

15. Dispositif d'administration selon la revendication 13 ou la revendication 14, **caractérisé en ce que,** lors de l'utilisation, le tube d'extraction (21 ; 27 ; 33) est positionné au-dessus de la nervure (6 ; 15 ; 22).

16. Dispositif d'administration selon l'une quelconque des revendications 10 à 15, **caractérisé en ce que** le dispositif d'administration est un inhalateur de poudre sèche.

17. Dispositif d'administration selon la revendication 16, **caractérisé en ce que** l'inhalateur comporte une multiplicité de réceptacles de médicament (1).

18. Dispositif d'administration selon la revendication 17, **caractérisé en ce que** chaque réceptacle (1) renferme deux médicaments.

19. Dispositif d'administration selon la revendication 18, **caractérisé en ce que** chaque médicament est maintenu de chaque côté de la nervure (6 ; 15 ; 22).

20. Réceptacle (1) selon l'une quelconque des revendications 1 a 8, **caractérisé en ce que** la poudre est choisie dans le groupe des médicaments pour le traitement de l'asthme, COPD ou infections respiratoires tels que des β₂-agonistes, par exemple fenoterol, formoterol, pirbuterol, reproterol, rimiterol, salbumatol, salmeterol et terbutaline ; des béta-stimulants tels que l'isoprenaline ; des bronchodilatateurs, par exemple theophylline, aminophylline et choline theophyllinate ; des anticholinergiques, par exemple du bromure d'ipratopium ; des stabilisateurs de cellule de masse, par exemple cromoglycate de sodium et cétotifèbe ; des agents anti-inflammatoires bronchiaux, par exemple nedocromil sodium ; et des stéroïdes, par exemple dipropionate de beclomethasone, fluticasone, budesonide, flunisolide et ciclesonide, et des isomères et/ou des sels ou des dérivés de ceux-ci.

21. Réceptacle (1) selon la revendication 20, **caractérisé en ce que** le réceptacle (1) comporte une combinaison de médicaments, choisis parmi les stéroïdes, telle que dipropionate de beclometasone et formoterol ; dipropionate de beclometasone et salmaterol ; fluticasone et formoterol ; fluticasone et salmaterol ; budesonide et formoterol ; budesonide et salmaterol ; flunisolide et formoterol ; et flunisolide et salmaterol.

22. Réceptacle (1) selon l'une quelconque des revendications 1 à 8, **caractérisé en ce que** le réceptacle (1) comporte un médicament actif systémique, tel que composés protéinés et/ou macromolécules, par exemple hormones et médiateurs, tels que insuline, hormone de croissance humaine, leuprolide et alpha interféron ; facteurs de croissance, anticoagulants, immunomodulateurs, cytokines et acides nucléides.
